Europäisches Patentamt

European Patent Office

Office européen des brevets

(19)

(11) **EP 0 778 045 B1**

(12) **EUROPEAN PATENT SPECIFICATION**

(45) Date of publication and mention
of the grant of the patent:
**20.02.2002   Bulletin 2002/08**

(51) Int Cl.⁷: **A61M 25/16**, B29C 55/30,
A61M 25/00

(21) Application number: **96119084.0**

(22) Date of filing: **28.11.1996**

(54) **Method of making an intravenous catheter and the cannula made by the method**

Herstellungsverfahren für einen Venenkatheter sowie Katheter nach diesem Verfahren

Méthode pour la fabrication d'un cathéter intraveineux et cathéter obtenu par cette méthode

(84) Designated Contracting States:
**DE FR GB IT**

(30) Priority: **04.12.1995   JP 31520295**

(43) Date of publication of application:
**11.06.1997   Bulletin 1997/24**

(73) Proprietor: **Nipro Corporation
Kita-ku, Osaka-shi (JP)**

(72) Inventors:
 • **Matsumoto, Takashi
   Tama-ku, Kawasaki-ku, Kanagawa-ken (JP)**
 • **Wakabayashi, Takahito
   Kusatsu-shi, Shiga-ken (JP)**
 • **Okada, Hiroshi
   Kusatsu-shi, Shiga-ken (JP)**

(74) Representative:
**Weisert, Annekäte, Dipl.-Ing. Dr.-Ing.
Patentanwälte Kraus & Weisert
Thomas-Wimmer-Ring 15
80539 München (DE)**

(56) References cited:
**EP-A- 0 508 118**

 • **PATENT ABSTRACTS OF JAPAN vol. 013, no.
   084 (C-572), 27 February 1989 & JP 63 270063 A
   (NITSUSHIYOO:KK), 8 November 1988, & JP 63
   270 063 A**

EP 0 778 045 B1

**Description**

**BACKGROUND OF THE INVENTION**

1.Field of the Invention

The present invention relates to a method of making an outer cannula for use to constitute an intravenous cannula, wherein a mold is used to form the end of a raw thin tube previously formed from a shape memory polyurethane resin.

2.Description of the Prior Art

Catheters (herein referred to as ' intravenous cannula ' ) have been and are used widely for transfusing blood or liquid medicines. The intravenous cannula will be kept in position in the blood vessels of patients, for a time necessary to the medical treatment. Generally, each interavenous cannula comprises an outer cannula made of a thermoplastic resin and an inner cannula of a stainless steel. The inner cannulae will be withdrawn from patients, after having cooperated with the outer cannulae to stick into the patients. Subsequently, blood transfusion set or infusion set, or the like will be connected with the outer cannulae dwelt in the patient bodies.

Distal end of outer cannulae have thin peripheral walls such that the difference in outside diameter between outer cannulae and the inner cannulae is diminished for a smoother piercing of patient skins. The materials forming the outer cannulae include PTFE ( polytetrafluoroethylene ), polturethane, polyethylene and the like.

In some cases, those outer cannulae with such thin ends are injection molded in its entirety. In other cases, one ends of previously prepared thin tubes are mechanically sharpened later or subsequently using a machining apparatus.

These prior art methods of forming the thin end of the outer cannulae are somewhat disadvantageous, particularly in a case wherein a shape memory polyurethane resin is used. It has been difficult for the injection molding process to produce thin outer cannulae in a rapid and efficient manner. The outer cannulae are likely to be damaged at their thin ends when and after ejected from a mold, and it is not easy to automatically arrange the ejected cannulae in a row. Thus, a labor and time consuming operation has been necessary to treat with the molded cannulae, despite the use of expensive molds. Inner cannulae varying largely in diameter will cause a significant variation in the engagement strength of them when fitting in the respective outer cannulae. In addition, save the hot runner type, the injection molds generally produce an amount of waste, causing a remarkable loss in material and further raising manufacture cost. On the other hand, the subsequent mechanical sharpening of the ends of previously produced tubes will inevitably cause them to be electrostatically charged during the process so that dusts and chips will stick more or less to the outer surface of each tube.

**SUMMARY OF THE INVENTION**

An object of the present invention made to resolve these problems is therefore to provide a novel method of making an outer cannula of an intravenous cannula, such that the outer cannula formed as a length of a tube made of a shape memory polyurethane resin dose have a thin distal end portion to reduce the penetration force of said cannula.

The present inventors have tried at first to directly make use of the prior art method as disclosed in JP-A-63270063 which discloses, apart from preheating the mold, the use of shape memory polyurethane and removing the tube from the mold after is has cooled the same method as defined in claim 1. However, they have found that the distal end of a shape memory polyurethane resin tube was highly susceptible to undesirably deformation if a mold was operated at an improper temperature. With an insert pin being inserted in an axial bore of the tube, an end thereof heated and softened in the mold did tend to elongate resulting in an undesired shape, when removing said tube from the mold together with the insert pin. Their best efforts to avoid these problems have led them to an improved method in which the mold must be heated to a temperature falling within a range of 120 °C to 180 °C , and the tube thus heated together with said insert pin and forced into a desired shape must then be cooled down before removed from the mold together with the insert pin.

The present method according to the invention is defined in claim 1.

Preferably, the outer cannula of the intravenous cannula thus manufactured by the method as summarized above has its distal end portion of a thickness of 0.05 mm or less.

Further embodiments are defined in claims 2 - 4.

The mold used in the method of the present invention preferably has a cavity comprising of a first tapered region located outermost, a second tapered region and a straight region located innermost. The first tapered region continuing from a tube insertion opening of the mold is tapered gently at a large angle, wherein the insertion opening is of a diameter sufficiently larger than the outside diameter of the tube. The second tapered region is tapered at a smaller angle than that of the first tapered region. The straight region above is of a diameter greater than a diameter of the insert pin but smaller than an outside diameter of the tube, as described above. An outer cannula may preferably be produced using this mold under a condition defined by the following two equations:

$$0.02 \leqq Dc - Dm \leqq 0.1 \qquad (i)$$

$$1.2\, t \leqq Dt - Dc \leqq 3t \qquad (ii)$$

wherein 'Dt' = outside diameter of the tube, 't' = thickness of the tube, 'Dm' = diameter of the insert pin, and 'Dc' = inside diameter of the mold's straight region, with all the dimensions being given in millimeters.

It is also desirable that the shape memory polyurethane resin has a solution viscosity of 0.47 or higher, and a breaking extention of 500 % or higher at a temperature of 110 °C.

## BRIEF DESCRIPTION OF THE DRAWINGS

Fig. 1 diagrammatically shows a method of making an outer cannula, in an embodiment of the present invention;

Fig. 2 illustrates a step included in the method, with some parts being shown in cross section;

Fig. 3 similarly illustrates another step, with the parts also shown in cross section;

Fig. 4a is a front elevation of an intravenous cannula comprising the outer cannula and an inner cannula, both partly shown in cross section;

Fig. 4b is a front elevation of the outer cannula;

Fig. 5 is a graph showing a relationship observed between the temperature at which a raw tube is processed and the solution viscosity of a polyurethane shape memory resin forming the raw tube, wherein an area giving preferable combinations of these parameters is denoted in the graph; and

Fig. 6 is another graph observed between the solution viscosity of said resin and breaking extention of said tube.

## DETAILED DESCRIPTION OF THE PREFERRED EMBODIMENTS

Now, some embodiments will be described referring to the drawings.

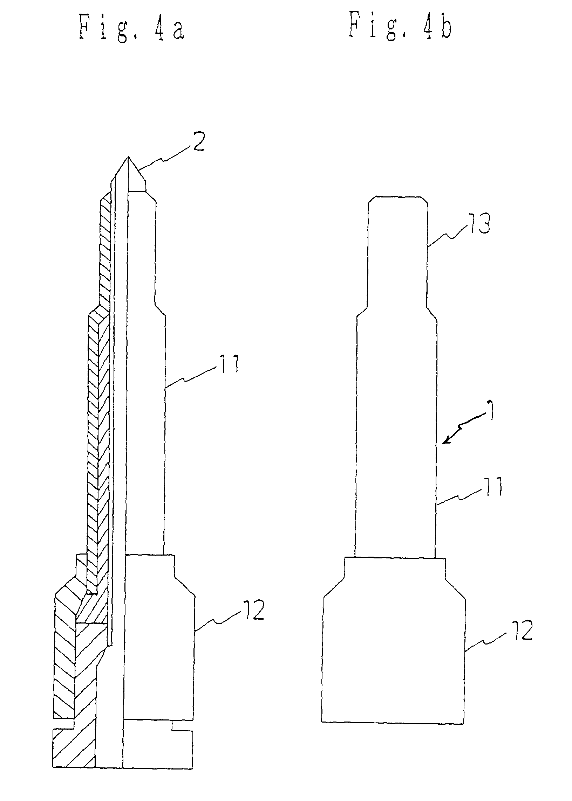

As will be seen in Figs. 4a and 4b, an outer cannula 1 of an intravenous cannula comprises a tube 11 and a hub 12. A finished distal end portion 13 of this tube has a peripheral wall whose thickness is 0.05 mm or less. Attached to a proximal end of the tube 11 is a hub 12 made of a plastics such as polypropylene, polyethylene or polycarbonate. The intravenous cannula is composed of the outer cannula 1 and an inner cannula 2 inserted therein. After this intravenous cannula 1 has pierced a patient's skin, the inner cannula 2 will be withdrawn so that a catheter or a guide wire (not shown) can be inserted through the outer cannula 1.

An end of a raw tube will be processed according to the present method so as to provide the outer cannula 1 with the finished distal end portion. In the present method, it can control appropriately the condition of molding such as configuration of cavity impression, a diameter of an insert pin 3, heating temperature, heating time, cooling temperature, cooling time and the like. Thus, the distal end portion can be finished to be of an accurate shape in an invariable and reliable manner.

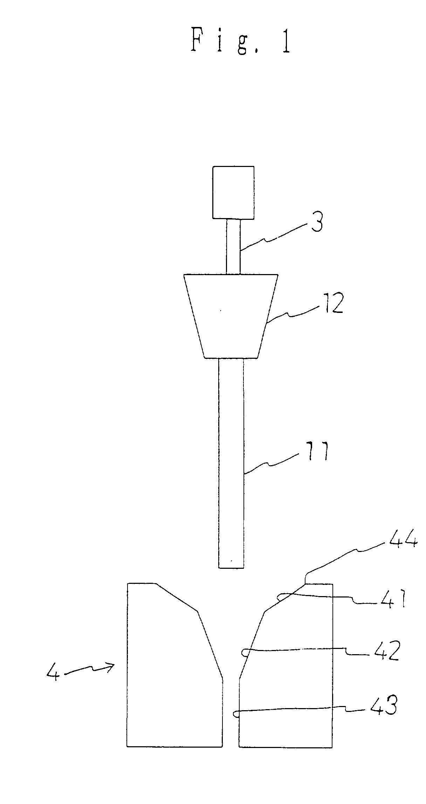

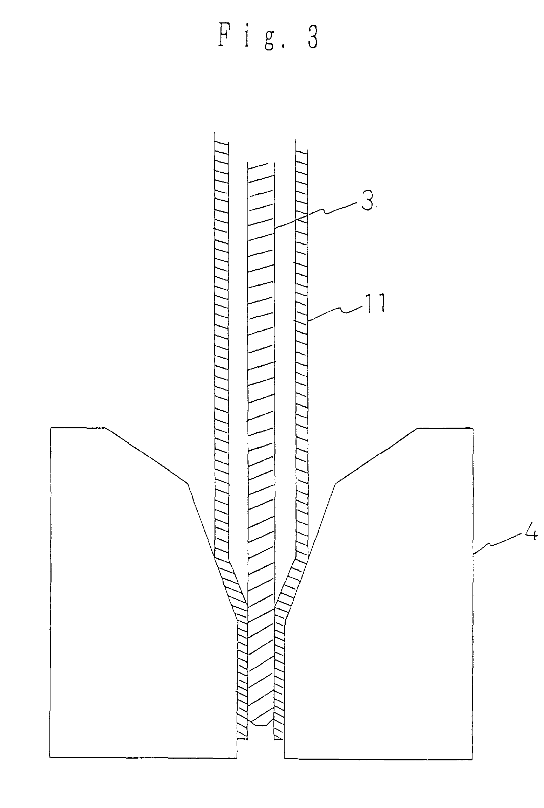

The method will now be described in detail, referring in particular Figs. 1 to 3.

At a first step, an outer cannula 1 substantially consisting of a tube 11 made of a shape memory polyurethane resin is prepared, with the tube 11 having an untreated distal end portion. An insert pin 3 having an outside diameter smaller than an inside diameter of the tube will then be inserted into the tube 11 such that the tip end of the insert pin 3 reaches a position retracted a predetermined distance properly remote from a distal end of the tube 11. The insert pin 3 has an outside diameter considerably smaller than an inside diameter of the tube 11. A special device not shown will be used to automatically insert the insert pin 3 into the tube 11. Save for a special case, the insert pin 3 is usually inserted into a position retracted a distance remote 4 - 6 mm, and more preferably about 5 mm from the distal end of the tube 11.

A mold 4 also prefered at the first step is illustrated in Fig. 1, and has a cavity composed of a first tapered region 41, a second tapered region 42 and a straigt region 43.

The first tapered region 41 has a cavity considerably greater diameter than outside diameter of the tube 11, and has a tube insertion opening 44 wide enough to smoothly receive said tube. An angle at which the first tapered region 41 is tapered may preferably be about 30 ° to 60 ° , though not delimited thereto.

The second tapered region 42 determining the shape of distal end portion of outer cannula 1 is gently tapered at a smaller angle so as to continue from the first tapered region 41 and to the straight tapered region 43. A diameter of the second tapered region 42 at its boundary to the first tapered region 41 is greater than the outside diameter of the tube 11, but is smaller than the same at its boundary to the straight region 43. The tapering angle of said second tapered region 42 may preferably be 4 ° to 15 ° . If this angle is significantly greater than 15 °, then the outer cannula 1 thus prepared will have a higher penetration force so that patients will suffer from pain when their veins are pierced with such an cannula.

If contrarily that angle is noticeably smaller than 4 ° , then the taper of the tube 11 will extend an excessive distance, thereby making it difficult to handle because inner cannula 2 will firmly engage to the outer cannula 1. Such an elongated distal end portion 13 of outer cannula 1 is excessively thin so that it will probably be bent or split when piercing into the patient skin.

The inside diameter of the straight region 43 is smaller than outside diameter of the tube 11 but greater than diameter of the insert pin 3. Length of this region 43 will be designed depending on the size of outer cannula 1.

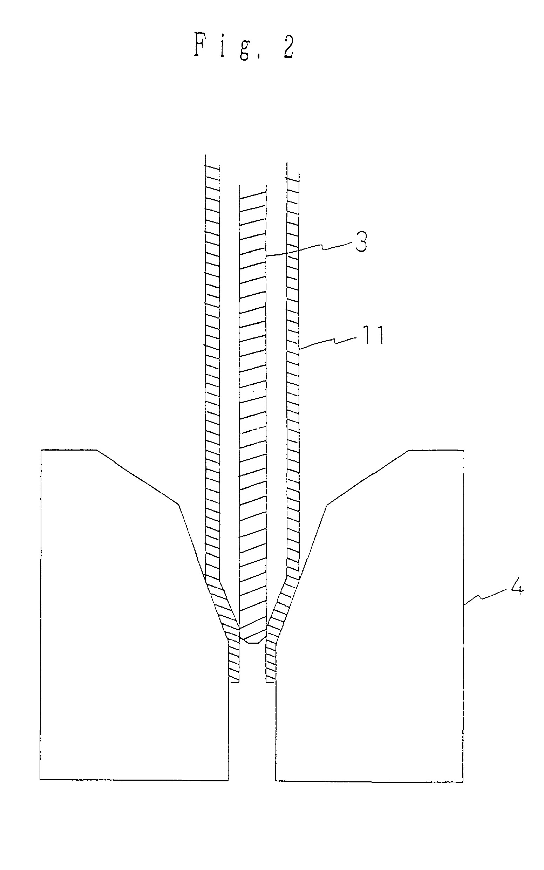

At a second step, with axially holding the hub 12 and the insert pin 3 so as not to move the insert pin 3 within the tube 11, the tube 11 having the insert pin 3 inserted therein at the first step will be inserted into the cavity formed in a mold 4 which is heated to a temperature of 120 °C to 180 °C until the insertion of the tube 11 into the cavity is obstructed. Then, as will be seen in Fig. 2, the insert pin 3 is driven within the tube 11 towards the extremity thereof until the tip end of insert pin 3 reaches a predetermined position of the mold 4. If the preheated mold 4 is colder than 120 °C , then the distal end portion of an outer cannula 1 of a finished product ( i.e., an intravenous cannula ) will undesirably expand to lose its regular shape when sterilizing said product at an elevated temperature. If the mold 4 is hotter than 180 °C , then fine corrugations will appear on the inside periphery of distal end portion 13 of tube 11, or its tapered portion will be collapsed or otherwise damaged. At a third step, the tube 11 is held in this state for 0.5 - 1 second to become sufficiently softened, subsequently the insert pin 3 is further driven within the tube towards the extremity thereof until the tip end of the insert pin 3 reaches a predetermined position of the mold 4, with holding the hub 12 of outer cannula 1. This state will be continued for 5 - 10 seconds. At a fourth step, the mold 4 will be cooled down by an air blow of about 20 °C for a period of 5 seconds. At a fifth step, the tube 11 will be removed from the mold 4 together with the insert pin 3 inserted therein. At a sixth step, the insert pin 3 will be pulled out of the tube. At a seventh step, the tube's distal end portion 13 is cut to be of a predetermined length.

Preferably, the mold 4 for forming the distal end of outer cannula 1 is of a shape defined by the following two equations:

$$0.02 \leqq Dc - Dm \leqq 0.1 \qquad (i)$$

$$1.2\,t \leqq Dt - Dc \leqq 3t \qquad (ii)$$

wherein ' ' Dt ' = outside diameter of the tube 11, ' t ' = thickness of the tube 11, ' Dm' = diameter of the insert pin 3, and ' Dc ' = inside diameter of the mold's straight region, with all the dimensions being given in millimeters.

If the difference: Dc - Dm in equation (i) is greater than 0.1, the outer cannula 1 will exert an undesirably strong penetration force when piercing the patient skin.

However a smaller difference than 0.02 will possibly cause the tube to be broken, and even if the process can be completed anyhow, the outer cannula 1 will tend in use to be bent or split when piercing said skin.

If the other difference: Dt - Dc in equation (ii) is greater than 3t, the tube 11 of outer cannula 1 will be excessively drawn or constricted in its outside diameter and thickness, thereby causing defects in the products. A longer heating time to avoid this problem will however raise higher production cost and lower manufacture efficiency.

With this difference Dt - Dc reduced below 1.2t, constriction of the tube wall will be insufficient, failing to provide a properly thinned distal end for said tube 11.

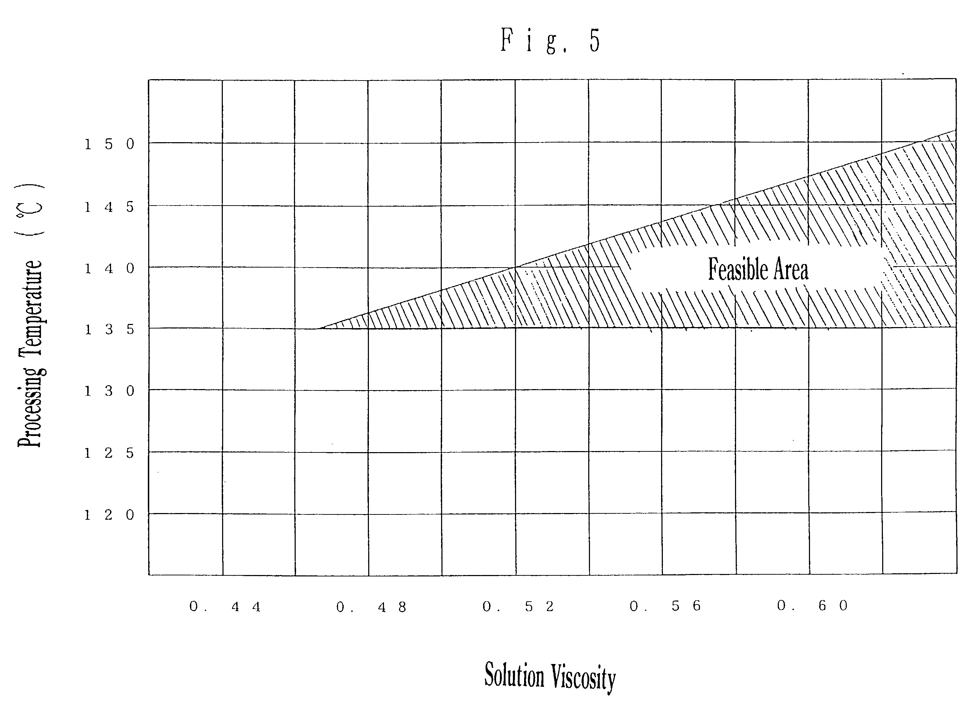

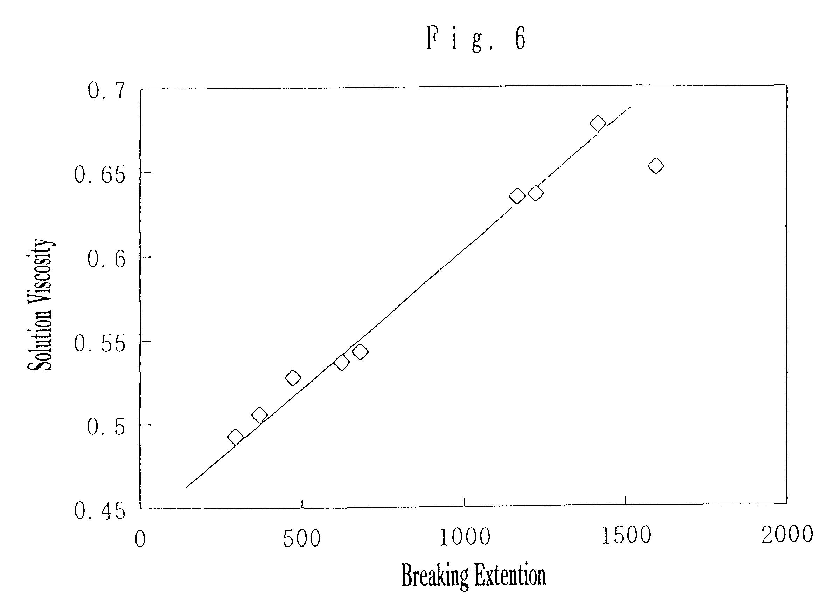

In examples of the embodiment discussed above, the polyurethane shape memory resin was a product from the Mitsubishi Heavy Industries Co., Ltd. This resin ' MM4510 ' of a polyester base was tested to give a relationship observed between its solution viscosity and the processing temperature as shown in Fig. 5, and another one between said viscosity and the breaking extention of products as shown in Fig. 6. As will be seen in these figures, it is preferable that the solution viscosity is 0,47 or higher and the breaking extention is 500 % or more at 110 °C.

It will now be apparent that the present method is useful in manufacture of the outer cannula of interavenous cannula that are of an accurate shape of the distal end portion and a lower penetration force to save patients from a piercing pain.

**Claims**

1. A method of making an outer cannula (1) for use to constitute intravenous cannula, the method comprising the steps of:

   preparing an outer cannula having a hub (12) and a tube (11) made of a shape memory polyurethane resin, wherein the tube (11) has an untreated distal end portion,
   inserting an insert pin (3) having a diameter smaller than an inside diameter of the tube (11) into the tube (11) until a tip end of the insert pin (3) reaches a first position retracted a predetermined distance remote from an extremity of a distal end of the tube (11) ;
   preheating a mold (4) to a temperature of 120°C to 180°C;
   holding with said first predetermined position of the insert pin (3) in said tube (11), inserting the tube (11) and the insert pin (3) into a cavity in said preheated mold (4) until the inserting of the tube (11) into the cavity is obstructed by a portion of a second tapered region of the cavity having a diameter greater than a diamater of the insert pin (3) and smaller than an outside diameter of the tube (11), and subsequently moving the insert pin (3) within the tube (11) towards the extremity thereof until the tip end of insert pin (3) reaches a second predetermined position in the mold cavity,
keeping the tube (11) at 120°C to 180°C for a certain period of time until the tube (11) is softened and thereafter, while holding a hub (12) of outer cannula (1), moving further the insert pin (3) within the tube (11) towards the extremity thereof until the tip end of the insert pin (3) reaches a third predetermined position in the mold cavity, and holding the insert pin (3) in the place for a certain period of time;
cooling the mold (4) for a certain period of time; after cooling the mold (4) removing the tube (11) from the mold (4) together with insert pin (3) inserted therein;
removing the insert pin (3) from the tube (11); cutting the distal end portion of the tube (11) to be of any desired length.

2. The method as defined in claim 1, wherein the cavity of the mold (4) comprises a first tapered region (41) located outermost, a second tapered region (42) and a straight region (43) located innermost, the first tapered region (41) continuing from a tube insertion opening (44) of the mold (4) is tapered gently at a large angle, wherein the insertion opening (44) is of a diameter sufficiently larger than the outside diameter of the tube (11), the second tapered region (42) is tapered at a smaller angle than that of the first tapered region (41), and the straight region (43) is of a diameter greater than the outside diameter of the insert pin (3) but smaller than that of the tube (11).

3. The method as defined in claim 1 or 2, wherein the mold satisfies conditions given by the following two equations:

$$0.02 \leq Dc - Dm \leq 0.1 \qquad (i)$$

$$1.2\, t \leq Dt - Dc \leq 3t \,. \qquad (ii)$$

wherein 'Dt' = outside diameter of the tube (11), 't' = thickness of the tube (11), 'Dm' = diameter of the insert pin (3), and 'Dc' = inside diameter of the mold's (4) straight region (43), with all the dimensions being given in millimeters.

4. The method as defined in claim 1, 2 or 3, wherein the shape memory polyurethane resin has a solution viscosity of 0.47 or higher and a breaking extention of 500% or more at 110°C.

**Patentansprüche**

1. Verfahren zum Herstellen einer äußeren Kanüle (1) für die Verwendung zum Bilden einer intravenösen Kanüle, wobei das Verfahren die folgenden Schritte umfasst:

Herstellen einer äußeren Kanüle, die ein Verbindungsstück (12) und ein aus Formgedächtnis-Polyurethanharz hergestelltes Rohr (11) hat, wobei das Rohr (11) einen unbehandelten distalen Endteil hat,

Einsetzen eines Einfügungsstifts (3), der einen Durchmesser hat, welcher kleiner als ein Innendurchmesser des Rohrs (11) ist, in das Rohr (11), bis ein Spitzenende des Einfügungsstifts (3) eine erste Position erreicht, die eine vorbestimmte Strecke entfernt von einem äußersten Endes eines distalen Endes des Rohrs (11) zurückgezogen ist;

Vorerhitzen einer Form (4) auf eine Temperatur von 120°C bis 180°C;

Beibehalten der genannten ersten vorbestimmten Position des Einfügungsstifts (3) in dem Rohr (11), Einsetzen des Rohrs (11) und des Einfügungsstifts (3) in einen Hohlraum in der vorerhitzten Form (4), bis das Einsetzen des Rohrs (11) in den Hohlraum durch einen Teil eines zweiten sich verjüngenden Bereichs des Hohlraums, der einen Durchmesser hat, welcher größer als ein Durchmesser des Einfügungsstifts (3) und kleiner als ein Außendurchmesser des Rohrs (11) ist, blokkiert wird, und nachfolgendes Bewegen des Einfügungsstifts (3) innerhalb des Rohrs (11) nach dem äußersten Endes desselben zu, bis das Spitzenende des Einfügungsstifts (3) eine zweite vorbestimmte Position in dem Formhohlraum erreicht,

Halten des Rohrs (11) auf 120°C bis 180°C während einer gewissen Zeitdauer, bis das Rohr (11) erweicht ist und danach, während man ein Verbindungsstück (12) der äußeren Kanüle (11) hält, weiteres Bewegen des Einfügungsstifts (3) innerhalb des Rohrs (11) nach dem äußersten Ende desselben zu, bis das Spitzenende des Einfügungsstifts (3) eine dritte vorbestimmte Position in dem Formhohlraum erreicht, und Halten des Einfügungsstifts (3) an Ort und Stelle während einer gewissen Zeitdauer;

Kühlen der Form (4) während einer gewissen Zeitdauer;

nach dem Kühlen der Form (4) Entfernen des Rohrs (11) aus der Form (4) zusammen mit dem darin eingesetzten Einfügungsstift (3);

Entfernen des Einfügungsstifts (3) aus dem Rohr (11);

Zuschneiden des distalen Endteils des Rohrs (11) so, dass es von irgendeiner gewünschten Länge ist.

2. Verfahren nach Anspruch 1, worin der Hohlraum der Form (4) einen ersten sich verjüngenden Bereich (41) umfasst, der am weitesten außen lokalisiert ist, einen zweiten sich verjüngenden Bereich (42) und einen geraden Bereich (43), der am weitesten innen lokalisiert ist, wobei der erste sich verjüngende Bereich (41), der von einer Rohreinführungsöffnung (44) der Form (4) weitergeht, mäßig unter einem großen Winkel verjüngt ist, worin die Einführungsöffnung (44) von einem Durchmesser ist, der genügend größer als der Außendurchmesser des Rohrs (11) ist, wobei sich der zweite, sich verjüngende Bereich (42) unter einem kleineren Winkel als jener des ersten sich verjüngenden Bereichs (41) verjüngt, und wobei der gerade Bereich (43) von einem Durchmesser ist, welcher größer als der Außendurchmesser des Einfügungsstifts (3), aber kleiner als jener des Rohrs (11) ist.

3. Verfahren nach Anspruch 1 oder 2, worin die Form die durch die folgenden beiden Gleichungen gegebenen Bedingungen erfüllt:

$$0,02 \leq Dc - Dm \leq 0,1 \qquad (i)$$

$$1,2\, t \leq Dt - Dc \leq 3t . \qquad (ii)$$

worin "Dt" = Außendurchmesser des Rohrs (11), "t" = Dicke des Rohrs (11), "Dm" = Durchmesser des Einfügungsstifts (3) und "Dc" = Innendurchmesser des geraden Bereichs (43) der Form (4), wobei alle die Dimensionen in Millimetern angegeben sind.

4. Verfahren nach Anspruch 1, 2 oder 3, worin das Formgedächtnis-Polyurethanharz eine Lösungsviskosität von 0,47 oder höher und eine Bruchdehnung von 500% oder mehr bei 110°C hat.

**Revendications**

1. Méthode de fabrication d'une canule extérieure (1) utilisable pour constituer un cathéter intraveineux, la méthode comprenant les étapes de :

préparation d'une canule extérieure ayant un manchon (12) et un tube (11) en résine polyuréthane à mémoire de forme, le tube (11) ayant une partie d'extrémité distale non traitée,
insertion d'une tige d'insert (3), ayant un diamètre plus petit qu'un diamètre intérieur du tube (11), dans le tube (11) jusqu'à ce qu'un bout de la tige d'insert (3) atteigne une première position en retrait à une distance prédéterminée d'une extrémité distale du tube (11),
préchauffage d'un moule (4) à une température de 120° à 180°C,
maintien de la dite première position prédéterminée de la tige d'insert (3) dans le dit tube (11),
insertion du tube (11) et de la tige d'insert (3) dans une cavité du dit moule préchauffé (4) jusqu'à ce que l'insertion du tube (11) dans la cavité soit empêchée par une partie d'une deuxième région conique de la cavité ayant un diamètre plus grand qu'un diamètre de la tige d'insert (3) et plus petit qu'un diamètre extérieur du tube (11), puis déplacement de la tige d'insert (3) à l'intérieur du tube (11) vers son extrémité jusqu'à ce que le bout de la tige d'insert (3) atteigne une deuxième position prédéterminée dans la cavité du moule,
maintien du tube (11) à une température de 120°C à 180C pendant un certain temps jusqu'à ce que le tube (11) soit ramolli et, ensuite, tout en saisissant le manchon (12) de la canule extérieure (1), déplacement de la tige d'insert (3) plus loin dans le tube (11) vers son extrémité jusqu'à ce que le bout de la tige d'insert (3) atteigne une troisième position prédéterminée dans la cavité du moule, et maintien de la tige d'insert (3) à cet endroit pendant un certain temps,
refroidissement du moule (4) pendant un certain temps,
après refroidissement du moule (4), enlèvement du tube (11) hors du moule (4) en même temps que la tige d'insert (3) insérée dans le tube,
enlèvement de la tige d'insert (3) hors du tube (11), et
coupe de la partie d'extrémité distale du tube (11) à toute longueur désirée.

2. Méthode selon la revendication 1, dans laquelle la cavité du moule (4) comprend une première région conique (41) la plus extérieure, une deuxième région conique (42) et une région cylindrique (43) la plus intérieure, la première région conique (41) s'étendant à partir d'un orifice d'insertion de tube (44) du moule (4) est de conicité très ouverte à grand angle, l'orifice d'insertion (44) est d'un diamètre suffisamment plus grand que le diamètre extérieur du tube (11), la deuxième région conique (42) a une conicité d'angle plus petit que celui de la première région conique (41), et la région cylindrique (43) a un diamètre plus grand que le diamètre extérieur de la tige d'insert (3) mais plus petit que celui du tube (11).

3. Méthode selon la revendication 1 ou 2, dans laquelle le moule satisfait aux conditions fixées par les deux équations suivantes :

$$0,02 \leq Dc - Dm \leq 0,1 \qquad \text{(i)}$$

$$1,2t \leq Dt - Dc \leq 3t \qquad \text{(ii)}$$

dans lesquelles Dt est le diamètre extérieur du tube (11), t est l'épaisseur du tube (11), Dm est le diamètre de la tige d'insert (3) et Dc est le diamètre intérieur de la région cylindrique (43) du moule (4), toutes les dimensions étant données en millimètres.

4. Méthode selon la revendication 1, 2 ou 3, dans laquelle la résine polyuréthane à mémoire de forme a une viscosité en solution de 0,47 ou plus et un allongement à la rupture de 500% ou plus, à 110°C.

# Fig. 1

Fig. 2

Fig. 3

3.

11

4

Fig. 4a      Fig. 4b

# Fig. 5

Processing Temperature (℃)

1 5 0

1 4 5

1 4 0

1 3 5

1 3 0

1 2 5

1 2 0

Feasible Area

0. 4 4      0. 4 8      0. 5 2      0. 5 6      0. 6 0

Solution Viscosity

# Fig. 6

EP 0 778 045 B1